Europäisches Patentamt

European Patent Office

Office européen des brevets

(19)

(11) **EP 1 099 408 A2**

(12) **EUROPÄISCHE PATENTANMELDUNG**

(43) Veröffentlichungstag:
**16.05.2001 Patentblatt 2001/20**

(51) Int Cl.⁷: **A61B 5/12**

(21) Anmeldenummer: **00124487.0**

(22) Anmeldetag: **09.11.2000**

(84) Benannte Vertragsstaaten:
**AT BE CH CY DE DK ES FI FR GB GR IE IT LI LU
MC NL PT SE TR**
Benannte Erstreckungsstaaten:
**AL LT LV MK RO SI**

(30) Priorität: **13.11.1999 DE 19954666**

(71) Anmelder: **Pilot Blankenfelde
medizinisch-elektronische Geräte GmbH
15827 Blankenfelde (DE)**

(72) Erfinder:
• **Stürzebecher, Ekkehard, Prof.Dr.-Ing. habil.
60596 Frankfurt am Main (DE)**
• **Cebulla, Mario, Dipl.-Ing.
60320 Frankfurt am Main (DE)**
• **Baag, Matthias
15827 Blankenfelde (DE)**
• **Thie, Rainer
15827 Blankenfelde (DE)**

(74) Vertreter: **Heyner, Klaus, Dr.-Ing.
Mittelweg 1h
01728 Bannewitz/Dresden (DE)**

(54) **Verfahren zur objektiven frequenzspezifischen Hörschwellenbestimmung mittels der Amplitude Modulation Following Response (AMFR)**

(57) Beschrieben wird ein Verfahren zur objektiven frequenzspezifischen Hörschwellenbestimmung mittels der Amplitude Modulation Following Response (AMFR) zur Anwendung insbesondere bei Säuglingen und Kleinkindern.

Die Konzeption der Erfindung besteht darin, anstelle eines einzelnen amplitudenmodulierten Trägers mehrere Träger, vorzugsweise 3 oder 5 Träger, deren Frequenz jeweils um eine Frequenzdifferenz $\Delta F$, vorzugsweise $\Delta F = F_{Mod}$ oder $\Delta F = 2 F_{Mod}$, versetzt ist, wobei alle Träger mit der gleichen Modulationsfrequenz $F_{Mod}$ bei einem Modulationshub zwischen 30% und 100%, vorzugsweise 100%, moduliert sind, als Reiz zu verwenden. Zusätzlich kann einer oder mehrere der amplitudenmodulierten Träger, bei ungerader Trägeranzahl vorzugsweise die zwischen den beiden äußeren Trägern liegenden Träger, frequenzmoduliert werden, wobei ein Frequenzhub zwischen 0% und 30%, vorzugsweise 20% gewählt wird. Bei den vorzugsweise verwendeten Parametern wird die Frequenzspezifität der Messung durch die spektrale Breite des Reizes noch nicht wesentlich eingeschränkt.

Im Unterschied zum üblichen Vorgehen, mit einem einzelnen statistischen Test die Entscheidung "AMFR vorhanden/nicht vorhanden" zu fällen, wird vorgeschlagen, vier verschiedene statistische Testverfahren (z.B. ) auf die Daten anzuwenden und das Vorliegen einer AMFR anzunehmen, sobald zwei der Tests Signifikanz anzeigen.

Die Hörschwellenbestimmung kann entweder vollautomatisch ablaufen oder vom Untersucher gesteuert werden.

Bei der Steuerung durch den Untersucher wird vorgeschlagen, die PC-Oberflächen für die Auswahl der Reizparameter und für die Ergebnisdarstellung zu verbinden.

FIG. 2.1

FIG. 2.2

**Beschreibung**

Die Erfindung betrifft das Gebiet der objektiven, d.h. von der Mitarbeit des Patienten unabhängigen Bestimmung der Hörschwelle unter Nutzung von akustisch evozierten Potentialen (AEP). Die objektive Hörprüfung wird vor allem bei Säuglingen und Kleinkindern angewendet, da in diesem Alter die sonst übliche subjektive Audiometrie, die eine aktive Mitarbeit des Patienten erfordert, noch nicht möglich ist.

Bekannt ist die automatische Bestimmung des Hörvermögens mit Klick-Reizen nach Patent DE 195 48 982.9 A1. Bei Klick-Reizung erhält man nur eine Summenantwort der frühen akustisch evozierten Potentiale (FAEP) aus dem Frequenzbereich von etwa 1000 Hz - 8000 Hz. Eine frequenzspezifische Hörschwellenbestimmung ist damit also nicht möglich.

Bekannt ist weiterhin die frequenzspezifische Hörschwellenbestimmung auf der Grundlage von Tonpuls-evozierten FAEP bei zusätzlicher Notched-Noise-Maskierung (Stürzebecher E, Wagner H, Cebulla M, Heine S, Jerzynski P. Rationelle objektive Hörschwellenbestimmung mittels Tonpuls-BERA mit Notched-Noise-Maskierung. Audiologische Akustik 1993;32:164-176). Bei diesem Verfahren ist aber bislang nur die Datengewinnung objektiv, die Auswertung der Registrierung (Entscheidung: Antwort vorhanden/nicht vorhanden) muß dagegen vom Untersucher durchgeführt werden. Das Problem der objektiven Auswertung ist nicht gelöst. Eine automatische Steuerung des gesamten Untersuchungsablaufs ist deshalb gegenwärtig nicht möglich. Weitere Nachteile sind: lange Untersuchungsdauer, u. U. starke Geräuschbelästigung durch das Maskierungsrauschen.

Ein Verfahren zur Hörschwellenbestimmung unter Nutzung der Amplitude Modulation Following Response (AMFR) ist aus Cohen LT, Rickards FW, Clark GM. A comparison of steady-stateevoked potentials to modulated tones in awake and sleepinh humans. J Acoust Soc Am 1991;90:2467-2479 und Griffiths SK, Chambers RD. The amplitude modulation-following response as an audiometric tool. Ear Hear 1991;12:235-241 bekannt.

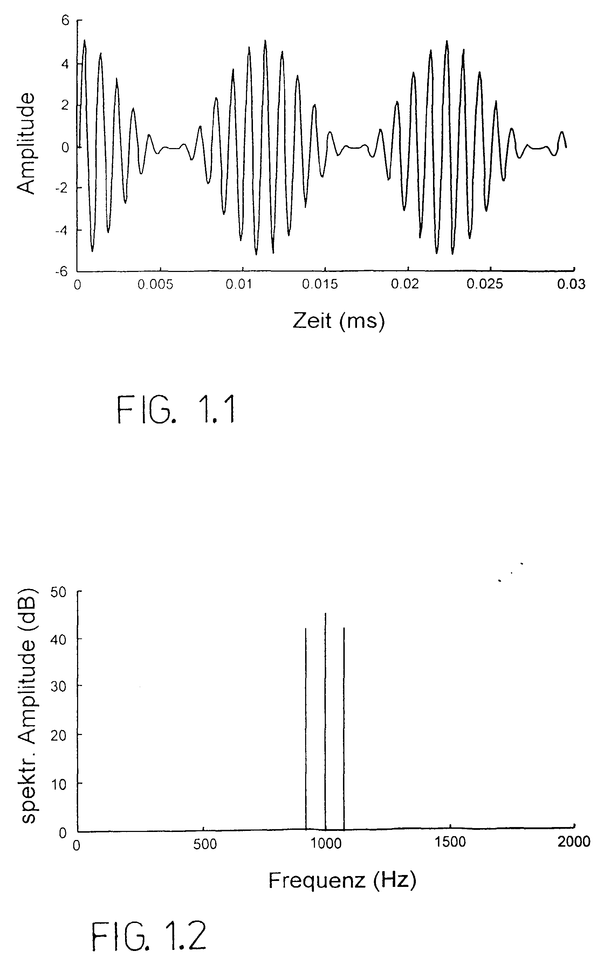

Die AMFR ist eine sogenannte "steady state response". Bei der AMFR-Gewinnung werden nicht, wie sonst üblich, in schneller Folge kurze akustische Reize (Tonpulse, Klicks) appliziert, sondern es wird mit einem kontinuierlichen amplitudenmodulierten Ton gereizt. Die Frequenz des modulierten Tones wird als Trägerfrequenz ($F_{Tr}$), die Frequenz des Modulationssignals als Modulationsfrequenz ($F_{Mod}$) bezeichnet. Fig. 1.1 zeigt das 1000-Hz-Reizsignal im Zeitbereich, Fig. 1.2 im Spektralbereich.

Die Prüfung des Hörvermögens erfolgt im Bereich $F_{Tr} \pm F_{Mod}$. Als Antwort erhält man ein kontinuierliches sinusförmiges Signal, dessen Frequenz der Modulationsfrequenz entspricht.

Die Auswertung der AMFR erfolgt im Spektralbereich.

Die Hörprüfung mittels AMFR hat folgende Vorteile gegenüber der üblichen Hörprüfung mittels der FAEP:

* Die Reizantwort ist ohne zusätzliche Maskierung frequenzspezifisch.
* Während die üblicherweise verwendeten FAEP durch ein breites und sehr variables Spektrum charakterisiert sind, ist die AMFR auf eine einzelne Spektrallinie beschränkt. Ein objektiver statistischer Nachweis der AMFR ist deshalb wesentlich einfacher als bei den übrigen AEP.
* In der Regel wird bei akustischer Reizung über Kopfhörer bei der EEG-Ableitung ein elektrischer Reizartefakt miterfaßt, der bei den üblichen AEP weder im Zeitbereich noch im Spektralbereich von der physiologischen Antwort sicher zu trennen ist. Bei der AMFR-Ableitung verursachen der Träger und die beiden Seitenfrequenzen im Abstand der Modulationsfrequenz einen elektrischen Artefakt. Da die Antwort auf eine einzelne Spektrallinie beschränkt ist, die nicht im Frequenzbereich des Trägers liegt, ist eine sichere Trennung von Antwort und Reizartefakt problemlos möglich.
* Es ist eine simultane Prüfung bei mehreren Frequenzen (Lins OG, Picton TW. Auditory steady state responses to multiple simultaneous stimuli. Electroenceph Clin Neurophysiol 1995;96: 420-432) und dadurch eine kürzere Untersuchungsdauer als bei der Notched-Noise BERA möglich.

Als Nachteile der bekannten Lösungen sind zu nennen:

Die Amplitude der Reizantwort liegt im Nanovolt-Bereich, ist also sehr klein. Die AMFR ist außerdem immer mit Rauschen (EEG) überlagert. Das Signal-Rausch-Verhältnis (SNR) ist sehr gering. Das gilt insbesondere in der Nähe der Hörschwelle, da mit abnehmendem Reizpegel die Amplitude der Antwort geringer wird. Hinzu kommt eine relativ große interindividuelle Streubreite der Antwortamplitude. Daraus ergibt sich auch eine entsprechende Streubreite der Nachweisbarkeit der Antwort in Schwellennähe. Für eine praktische Anwendung ist diese Streuung zu groß. Um eine größere Antwortamplitude zu erzielen, wurde von Cohen et al. (1991) ein frequenzmodulierter Träger zusätzlich amplitudenmoduliert. Der Amplitudengewinn gegenüber der einfachen Amplitudenmodulation ist aber nicht ausreichend. Ein kommerzielles Gerät zur objektiven Hörschwellenbestimmung auf der Basis der AMFR ist bisher nicht bekannt.

Aufgabe der Erfindung ist es, unter Nutzung der Amplitude Modulation Following Response (AMFR) ein objektives frequenzspezifisches Hörprüfverfahren zu entwickeln, dass vollautomatisch arbeiten kann und eine zuverlässige Bestimmung der frequenzabhängigen Hörschwelle ermöglicht.

Eine erfindungsgemäße Lösung dieser Aufgabe ist im Patentanspruch 1 angegeben. Weiterbildungen der Erfindung sind in den Unteransprüchen gekennzeichnet. Bei der bekannten Stimulation mit einem amplitudenmodulierten Träger wird entsprechend der geringen spektralen Breite des Reizes ($F_{Tr} \pm F_{Mod}$) nur ein schmaler Bereich auf der Basilarmembran des Innenohres aktiviert. Die Amplitude der abgeleiteten Summenantwort ist deshalb gering. Eine größere Antwortamplitude ist zu erwarten, wenn die spektrale Breite des Reizes vergrößert wird, dabei aber realisiert wird, daß die Antwort weiterhin exakt auf eine einzelne Spektrallinie beschränkt bleibt.

Die Konzeption der Erfindung besteht deshalb darin, anstelle eines einzelnen amplitudenmodulierten Trägers mehrere Träger, vorzugsweise 3 oder 5 Träger, deren Frequenz jeweils um eine Frequenzdifferenz ΔF, vorzugsweise ΔF= $F_{Mod}$ oder ΔF = 2 $F_{Mod}$, versetzt ist, wobei alle Träger mit der gleichen Modulationsfrequenz $F_{Mod}$ bei einem Modulationshub zwischen 30% und 100%, vorzugsweise 100%, moduliert sind, als Reiz zu verwenden. Zusätzlich kann einer oder mehrere der amplitudenmodulierten Träger, bei ungerader Trägeranzahl vorzugsweise die zwischen den beiden äußeren Trägern liegenden Träger, frequenzmoduliert werden, wobei ein Frequenzhub zwischen 0% und 30%, vorzugsweise 20% gewählt wird.

Bei den vorzugsweise verwendeten Parametern wird die Frequenzspezifität der Messung durch die spektrale Breite des Reizes noch nicht wesentlich eingeschränkt.

Im Unterschied zum üblichen Vorgehen, mit einem einzelnen statistischen Test die Entscheidung "AMFR vorhanden/nicht vorhanden" zu fällen, wird vorgeschlagen, mehrere statistische Testverfahren, vorzugsweise vier, auf die Daten anzuwenden und das Vorliegen einer AMFR anzunehmen, sobald wenigstens zwei der Tests Signifikanz anzeigen.

Die Hörschwellenbestimmung kann entweder vollautomatisch ablaufen oder vom Untersucher gesteuert werden.

Für die vom Untersucher gesteuerte Hörschwellenbestimmung wird vorgeschlagen, die bei bekannten Lösungen separaten PC-Oberflächen für die Reizauswahl (Frequenz, Reizpegel) und für die Ergebnisdarstellung (Audiogramm) auf folgende Weise zu verbinden: Auf dem PC-Bildschirm wird je ein Audiogrammformular (DIN-Norm) für das rechte und das linke Ohr bzw. umschaltbar für das rechte oder linke Ohr dargestellt.

Die Auswahl eines akustischen Reizes einer bestimmten Frequenz mit einem bestimmten Pegel erfolgt durch Mausklick auf den Kreuzungspunkt der vertikalen Rasterlinie für die auf der Abszisse markierte Frequenz und der horizontalen Rasterlinie für den auf der Ordinate markierten Reizpegel. Alternativ ist die Anwahl des Kreuzungspunktes über die Pfeiltasten der PC-Tastatur möglich. Bei simultaner Hörprüfung bei mehreren Prüffrequenzen (Lins und Picton, 1995) wird in gleicher Weise ein zweites und ggf. ein drittes Reizparameter-Paar vorgewählt. Nach dem Start der Messung und der statistischen Testung wird das Ergebnis (Reizantwort vorhanden/nicht vorhanden) im Audiogrammformular am Kreuzungspunkt von geprüfter Frequenz und Reizpegel so markiert, daß erkennbar ist, ob bei den gewählten Reizparametern eine Antwort nachweisbar ist oder nicht.

Die Anwendung der erfindungsgemäßen Lösung hat folgende Vorteile:

- Die Breite des Reizspektrums ist exakt einstellbar.
- Das Spektrum ist nahezu rechteckförmig, d.h. die das Spektrum bildenden Spektrallinien haben alle nahezu die gleiche Amplitude.
- Die Antwort ist weiterhin exakt auf die Modulationsfrequenz beschränkt.
- Das die Antwortamplitude charakterisierende SNR ist im Mittel um den Faktor 2 größer als bei der herkömmlichen Stimulation mit einem einzelnen amplitudenmodulierten Träger und auch größer als bei zusätzlicher Frequenzmodulation des einzelnen amplitudenmodulierten Trägers. Die bei herkömmlicher Stimulation resultierende Streubreite der Differenz zwischen der subjektiv und der objektiv bestimmten Hörschwelle wird dadurch deutlich reduziert, so daß ein praktischer Einsatz der objektiven Hörprüfmethode ermöglicht wird.
- Die Anwendung von bekannten statistischen Testverfahren für den AMFR-Nachweis ermöglicht die Realisierung einer vollautomatischen objektiven frequenzspezifischen Hörprüfung.
- Bei der vorgeschlagenen Lösung, statt mit einem einzelnen Test die Entscheidung "AMFR vorhanden/nicht vorhanden" zu fällen, 4 verschiedene statistische Testverfahren (z.B. ) auf die Daten anzuwenden und das Vorliegen einer AMFR anzunehmen, sobald 2 der Tests Signifikanz anzeigen, wird eine geringere Wahrscheinlichkeit für falsch-positive Testergebnisse erreicht.
- Bei Anwendung der erfindungsgemäßen Reizkonfiguration kann ohne Einschränkung auch die von Lins & Picton (1995) vorgeschlagene simultane Hörprüfung bei mehreren unterschiedlichen Prüf-Frequenzen durchgeführt werden.
- Abgestimmt auf den zu untersuchenden Frequenzbereich kann eine unterschiedliche spektrale Breite des Reizes eingestellt werden.
- Durch die Verbindung von Reizparameter-Wahl und Ergebnisdarstellung ist ein Wechsel zwischen Ergebnisdarstellung (Entscheidung über die als nächstes zu wählenden Reizparameter) und dem Menü für die Reizparameter-Wahl nicht mehr erforderlich. Dadurch werden Irrtümer bei der Reizparameter-Wahl, insbesondere bei simultaner Hörprüfung bei mehreren Frequenzen vermieden.
- Bei der vorgeschlagenen Verbindung von Reizparameter-Wahl und Ergebnisdarstellung werden beide Reizparameter Frequenz und Reizpegel mit einem Mausklick gewählt. Aus der grafischen Ergebnisdarstellung ist unmittelbar erkennbar, welche Reizparameter für den nächsten Untersuchungsschritt zu wählen sind.

Weitere Einzelheiten, Merkmale und Vorteile der Erfindung ergeben sich aus der nachfolgenden Beschreibung eines Ausführungsbeispiels unter Bezugnahme auf die zugehörige Zeichnung. Es zeigen

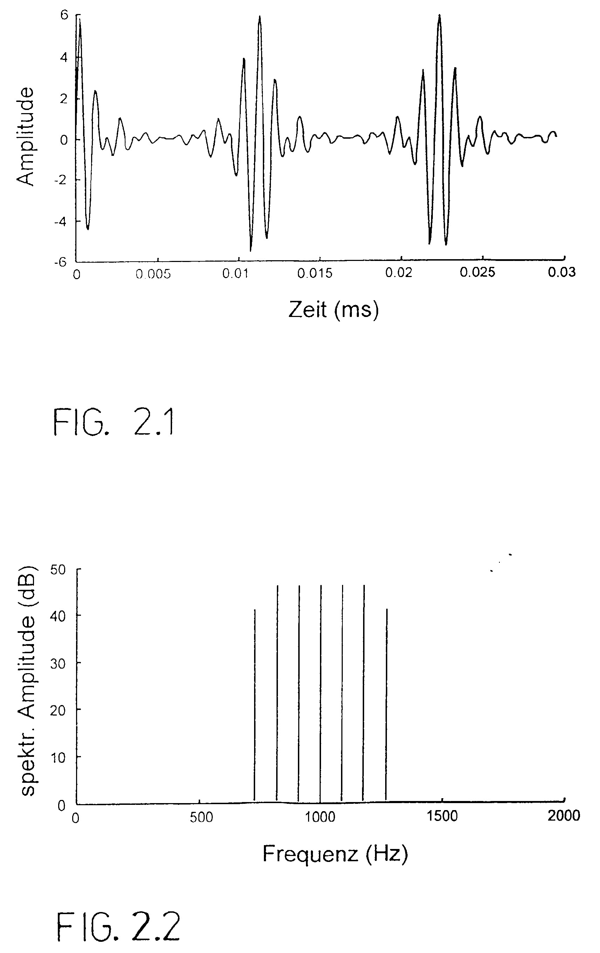

Fig. 2.1   die Zeitfunktion des akustischen Reizes als Überlagerung von drei amplitudenmodulierten Trägern im Abstand von 2 $F_{Mod}$ und

Fig. 2.2   das Spektrum der in Fig. 2.1 dargestellten Zeitfunktion.

Bei einem Kleinkind, bei dem der Verdacht auf eine hochgradige Hörstörung besteht, soll, da die üblichen subjektiven Verfahren in diesem Alter nicht zu einem ausreichend genauen Ergebnis führen, objektiv mittels der AMFR die frequenzspezifische Hörschwelle ermittelt werden, um auf dieser Basis die Versorgung mit einem Hörgerät oder ggf. auch mit einem Cochlear Implant durchführen zu können.

Die Untersuchung erfolgt während des natürlichen Schlafs oder unter Sedierung. Die Hörprüfung erfolgt bei den Frequenzen 250 Hz, 500 Hz, 1000 Hz, 2000 Hz und 4000 Hz.

Die Zusammensetzung der akustischen Stimuli wird am Beispiel des Reizes für den 1000-Hz-Frequenzbereich erläutert:

Die Modulationsfrequenz ist $F_{Mod}$ = 89,84375 Hz. Der akustische Reiz besteht aus drei Trägern, deren Frequenz jeweils um die Frequenzdifferenz $\Delta F = 2\,F_{Mod}$, versetzt ist:

$$F_{Tr0} = 988,28 \text{ Hz}$$

$$F_{Tr1} = F_{Tr0} + 2\,F_{Mod} = 1167,9675 \text{ Hz}$$

$$F_{Tr2} = F_{Tr0} - 2\,F_{Mod} = 808,5925 \text{ Hz}$$

Der mittlere Träger ist frequenz- und amplitudenmoduliert, der Frequenzhub beträgt 20%, der Amplitudenhub 100%. Frequenz- und Amplitudenmodulation sind gleichphasig. Die nicht ganzzahligen Frequenzen ergeben sich durch die Forderung, daß die Periodendauer der Modulationsfrequenz ein ganzzahliges Vielfaches der Periodendauer der Trägerfrequenzen sein soll. Als zweite Forderung muß erfüllt.sein, daß die Länge einer mittels Fourier Transformation (FT) transformierten Epoche des abgeleiteten EEG ein ganzzahliges Vielfaches der Periodendauer des Modulationssignals ist.

In Fig. 2.1 ist das Spektrum des Reizes, in Fig.

2.2 ein Ausschnitt aus der Zeitfunktion des Reizes ohne Frequenzmodulation des mittleren Trägers dargestellt.

Im Steuer-PC wird vor dem Start der Untersuchung das Stimulationssignal mit der Länge einer Epoche berechnet und im Speicher abgelegt. Der berechnete Reizabschnitt enthält nur ganzzahlige Vielfache der drei Trägerfrequenzen und der Modulationsfrequenz. Bei der akustischen Stimulation wird der Speicher zyklisch ausgelesen. Das dadurch kontinuierliche Signal wird D/A-konvertiert, verstärkt und mit dem gewählten Pegel (dBnHL) über Kopfhörer auf das Ohr des Patienten gegeben.

Während der akustischen Reizung wird das EEG mittels Klebeelektroden von der Kopfhaut abgeleitet. Die Elektrodenlage ist Vertex/ipsilaterales Mastoid, Erde: Stirn. Das EEG wird verstärkt und A/D-konvertiert. Der Takt für D/A-Konverter und A/D-Konvertierung muss synchronisiert sein. Anderenfalls ist nicht gewährleistet, daß eine digitalisierte Epoche des verstärkten EEG nur ganzzahlige Vielfache der Modulationsfrequenz und der Trägerfrenzen enthält, mit der Folge, daß bei der FT durch "angeschnittene" Perioden Seitenbänder beim Nutzsignal und beim Reizartefakt entstehen.

Für die weitere Beschreibung wird angenommen, daß die Abtastfrequenz von D/A- und A/D-Konverter gleich ist und 16 kHz beträgt. Das digitalisierte EEG wird während der Stimulation fortlaufend gespeichert. Eine Epoche wird durch 4096 Abtastwerte repräsentiert und hat eine Länge von 0,256 s. Parallel zur akustischen Reizung und Datenerfassung werden EEG-Abschnitte mit einer Länge von 8 Epochen (2,048 s), im weiteren als Sweeps bezeichnet, mittels FT in den Spektralbereich transformiert. Das für jeden Sweep berechnete Spektrum wird gespeichert. Die Frequenzauflösung im Spektrum beträgt 0,48828125 Hz (1/2,048 s).

Die Hörschwellenbestimmung wird im Anwendungsbeispiel vom Untersucher gesteuert. Das Vorgehen wird am Beispiel der Bestimmung der Hörschwelle des rechten Ohres bei 1000 Hz erläutert. Der Startreizpegel sei 60 dBnHL. Durch

Mausklick auf den Kreuzungspunkt 1000 Hz/60 dBnHL im Audiogrammformular auf dem Bildschirm werden die Reizparameter vorgewählt. Nach Bestätigung mit der Enter-Taste startet die Untersuchung. Sobald die Spektren von 20 Sweeps vorliegen, erfolgt die erste statistische Testung mit 4 Testverfahren. Jeweils nach Erhöhung des Stichprobenumfangs um 5 Sweeps wird erneut getestet. Solange das Testergebnis nicht bei mindestens 2 der 4 Tests signifikant ist, wird der Stichprobenumfang kontinuierlich vergrößert, bis eine Antwort nachgewiesen wird oder bis ein vorgegebener maximaler Stichprobenumfang (z.B. 50 Sweeps) erreicht ist. Hier sei angenommen, daß bei 1000 Hz/60 dBnHL auch mit 50 Sweeps keine Antwort nachweisbar ist. Im Audiogrammformular auf dem Bildschirm wird das entsprechend markiert, z.B. durch einen grauen Kreis im Kreuzungspunkt 1000 Hz/60 dBnHL. Der Untersucher erhöht daraufhin den Reizpegel auf z.B. 80 dBnHL durch Mausklick auf den Kreuzungspunkt 1000 Hz/80 dBnHL. Es sei angenommen, daß bei 80 dBnHL eine Antwort nachweisbar ist. Die Markierung erfolgt durch einen roten Kreis im Kreuzungspunkt 1000 Hz/80 dBnHL. Der Untersucher wählt nun einen Reizpegel von 70 dBnHL. Es sei angenommen, daß bei diesem Reizpegel keine Antwort nachweisbar ist. Im Audiogrammformular wird der Kreuzungspunkt 1000 Hz/70 dBnHL durch einen grauen Kreis markiert. Die Hörschwelle wird bei 80 dB angenommen.

In gleicher Weise erfolgt die Untersuchung bei den Frequenzen 250 Hz, 500 Hz, 2000 Hz und 4000 Hz, ggf. simultan bei 2 oder 3 Frequenzen, wobei der Abstand 2 Oktaven betragen sollte. Wurde bei einer Frequenz bei mehreren Reizpegeln eine Antwort nachgewiesen, wird von diesen nur der Kreuzungspunkt für den niedrigsten Reizpegel mit einem roten Kreis markiert, die anderen z.B. mit einem rosa Kreis. Die bei den 5 Frequenzen mit einem roten Kreis markierten Kreuzungspunkte werden mit einem roten Linienzug (=Hörschwellenkurve) verbunden.

Wahlweise kann die Hörschwellenbestimmung für jede Frequenz einzeln oder gemäß dem Vorschlag von Lins und Picton (1995) simultan für mehrere Frequenzen erfolgen. Eine Prüfung in 5-dB-Schritten ist möglich, aber zeitaufwendiger, da bei jeder Frequenz eine weitere Untersuchung, hier im Beispiel bei 1000 Hz/75 dBnHL, erforderlich ist.

**Patentansprüche**

1.  Verfahren zur objektiven frequenzspezifischen Hörschwellenbestimmung mittels der Amplitude Modulation Following Response (AMFR) zur Anwendung insbesondere bei Säuglingen und Kleinkindern, **dadurch gekennzeichnet,** dass die für die akustische Stimulation verwendeten Reize aus mehreren Trägern bestehen, die alle mit der gleichen Modulationsfrequenz amplitudenmoduliert sind und deren Frequenzen um eine Differenz ΔF gegeneinander versetzt sind, wobei für den objektiven Nachweis der AMFR mehrere statistische Verfahren eingesetzt werden und dass das Vorliegen einer AMFR als nachgewiesen gilt, wenn mindestens zwei der Tests ein signifikantes Ergebnis liefern.

2.  Verfahren nach Anspruch 1, **dadurch gekennzeichnet,** dass die Träger um die einfache Modulationsfrequenz ΔF gegeneinander versetzt sind.

3.  Verfahren nach Anspruch 1, **dadurch gekennzeichnet,** dass die Träger um die doppelte Modulationsfrequenz ΔF gegeneinander versetzt sind.

4.  Verfahren nach einem der Ansprüche 1 bis 3, **dadurch gekennzeichnet,** dass die Reize aus drei oder fünf Trägern bestehen, wobei alle Träger bei gleicher Modulationsfrequenz einen Modulationshub (Amplitudenhub) zwischen 30% und 100% aufweisen.

5.  Verfahren nach einem der Ansprüche 1 bis 4, **dadurch gekennzeichnet,** dass zusätzlich einer oder mehrere Träger, bei einer ungeraden Trägeranzahl vorzugsweise die zwischen den beiden äußeren Trägern liegenden Träger, frequenz- und amplitudenmoduliert sind.

6.  Verfahren nach Anspruch 5, **dadurch gekennzeichnet,** dass ein Frequenzhub zwischen 0% und 30% gewählt wird.

7.  Verfahren nach einem der Ansprüche 1 bis 6, **dadurch gekennzeichnet,** dass für die Bestimmung der Hörschwelle bei den tiefen, mittleren und hohen Hörfrequenzen Reize mit unterschiedlicher Bandbreite eingesetzt werden, wobei die Bandbreite des Reizes durch die Anzahl der Träger und/oder durch die Differenz ΔF zwischen den Trägerfrequenzen veränderbar ist.

8.  Verfahren nach einem der Ansprüche 1 bis 7, **dadurch gekennzeichnet,** dass vier verschiedene statistische Verfahren eingesetzt werden und das Vorliegen einer AMFR als nachgewiesen gilt, wenn mindestens zwei der Tests ein signifikantes Ergebnis liefern.

9.  Verfahren nach einem der Ansprüche 1 bis 8, **dadurch gekennzeichnet,** dass die Ermittlung der Hörschwellenkurve wahlweise vom Untersucher oder vollautomatisch durch einen geeigneten Algorithmus gesteuert wird.

10. Verfahren nach einem der Ansprüche 1 bis 9, **dadurch gekennzeichnet,** dass bei der automatischen Ablaufsteuerung der Hörschwellenbestimmung der Startreizpegel nur für eine Frequenz vom Untersucher vorgegeben wird und die Startreizpegel für die übrigen Frequenzen adaptiv vom Steueralgorithmus gewählt werden, wobei die adaptive Vorgabe sich an den bereits ermittelten Hörschwellen, vorzugsweise an der Hörschwelle einer benachbarten Frequenz orientiert.

11. Verfahren nach einem der Ansprüche 1 bis 10, **dadurch gekennzeichnet,** dass bei dem vom Untersucher gesteuerten Ablauf die PC-Oberflächen für die Auswahl der Reizparameter (Frequenz und Reizpegel) und für die Ergebnisdarstellung (Audiogrammformular) verbunden sind und die Auswahl der Reizparameter im Audiogrammformular entweder durch einen Mausklick auf den Kreuzungspunkt der vertikalen Rasterlinie für die gewünschte Prüffrequenz und der horizontalen Rasterlinie für den gewünschten Reizpegel oder durch getrenntes Anklicken von Frequenz und Reizpegel erfolgt, wobei im letzteren Fall die Auswahl direkt auf der Ordinate und auf der Abszisse des Audiogrammformulars oder an Hand von geeigneten Darstellungen des verfügbaren Frequenz- und Reizpegelbereichs außerhalb des dargestellten Audiogrammformulars erfolgen kann.

12. Verfahren nach Anspruch 11,
**dadurch gekennzeichnet,** dass die Ergebnisdarstellung im Audiogrammformular so durchgeführt wird, daß erkennbar ist, ob bei einem Reizparameter-paar bereits eine Untersuchung durchgeführt wurde und ob dabei eine Antwort nachgewiesen wurde oder nicht, wobei bei jeder Frequenz der Kreuzungspunkt für den niedrigsten Reizpegel, bei dem eine Antwort nachgewiesen wurde, mit den für die Audiometrie üblichen Kennzeichen markiert wird und diese bei den verschiedenen Frequenzen so markierten Kreuzungspunkte durch einen Linienzug verbunden werden.

FIG. 1.1

FIG. 1.2

FIG. 2.1

FIG. 2.2